# EUROPEAN PATENT APPLICATION

(11) **EP 1 609 783 A1**
(43) Date of publication of application: **28.12.2005**
(21) Application number: 04076731.1
(22) Date of filing: 17.06.2004
(51) Int. Cl.: C07D 261/20, A61K 31/42

(54) **Marine compounds with calcium channel blocking properties for Alzheimer's disease treatment**

(71) Applicant: Neuropharma S.A.U., 28760 Tres Cantos (Madrid) (ES)
(72) Inventor: Martinez Gil, Ana, 28760 Tres Cantos (Madrid) (ES); Alonso Gordillo, Diana, 28760 Tres Cantos (Madrid) (ES); Dorronsoro Diaz, Isabel, 28760 Tres Cantos (Madrid) (ES); Garcia Palomero, Esther, 28760 Tres Cantos (Madrid) (ES); de Austria de Luque, Celia, 28760 Tres Cantos (Madrid) (ES); Usan Egea, Paola, 28760 Tres Cantos (Madrid) (ES); del Monte Millan, Maria, 28760 Tres Cantos (Madrid) (ES); Median Padilla, Miguel, 28760 Tres Cantos (Madrid) (ES)
(74) Representative: Portela Gasch, Sergio Raul

(57) **Abstract**

The invention provides compounds having two spiro heterocyclic units connected through a linker of a certain length. Some of these compounds have been obtained from *Aplysinia sp.* The compounds exhibit VDCC blocker activity as well as acetylcholinesterase and butyrylcholinesterase inhibition activities. Therefore they are useful in the treatment of use in the treatment of brain ischemia, stroke, cognitive disorders as senile dementia, cerebrovascular dementia, mild recognition impairment, attention deficit disorder, and/or neurodegenerative dementing disease with aberrant protein aggregations as specially Alzheimers's disease or condition, or prion disease as Creutzfeld-Jacob disease or Gerstmann-Straussler-Scheinher disease.

## Description

### FIELD OF THE INVENTION

This invention relates to compounds obtained from the marine organism *Aplysina sp*., and more particularly to spirocompounds isolated from the mediterranean sponge *Aplysina cavernicola*, and their use in the treatment of brain ischemia, stroke, cognitive disorders as senile dementia, cerebrovascular dementia, mild recognition impairment, attention deficit disorder, and/or neurodegenerative dementing disease with aberrant protein aggregations as specially Alzheimers's disease or condition, or prion disease as Creutzfeld-Jacob disease or Gerstmann-Straussler-Scheinher disease.

### BACKGROUND OF THE INVENTION

VDCC blockers (block voltage-dependent calcium channel) are traditionally used for treatment of cardiovascular diseases, but these drugs may also be applied for pain treatment or for some neurodegenerative disorders, for instance Alzheimer's disease (AD) (Verkhratsky et al., 2003, *J Cell Mol Med*, 7, 351-61).
AD is an irreversible disorder, characterized by a progressive loss of several cognitive abilities. Although many factors have been implicated in this type of dementia, its etiology and pathogenesis remains unclear. One of the characteristic changes in the AD brain is the deficit of cerebral acetylcholine. The "cholinergic hypothesis" (Winkler et al., 1998, *J Mol Med,* 76, 555-67) represents one of the most useful approaches involved in treatment of AD, and led to development of drugs with an AChE (acetylcholinesterase) inhibition activity. AChE expression is upregulated around amyloid plaques in AD patients, but this mechanism is unknown, although there are evidences suggesting that β-amyloid could increase AChE expression by increasing intracellular calcium involving the opening of VDCC (Sbema et al., 1997, *J Neurochem, 69,* 1177-84). There is also emerging evidence showing that an altered calcium homeostasis can play an important role in this pathology (Mattson et al., 2000, Trends Neurosci, 23, 222-9).

The oceans are the source of a large group of structurally unique natural products that are mainly accumulated in invertebrates such as sponges, tunicates, bryozoans, and mollusc (Burkhard et al., 2003, *Drug Dis Today*, 8, 2, 536-544). Several of these compounds show pronounced pharmacological activities and are interesting candidates for new drugs in several areas of treatment (Newman et al., 2003, *J Nat Prod,* 66, 1022-1037). Once again sponges have provided more marine natural products than any order phylum, due in part to their propensity to produce bioactive metabolites (Faulkner et al., 2002, *Nat Prod Rep,* 19, 1-48).

There is still a need for new compounds able to modulate VDCC with additional AChE and butyrylcholinesterase (BuChE) inhibitory activities, which could be useful in the treatment of cognitive or neurodegenerative diseases. With this aim, a screening of marine compounds was performed on SH-SY5Y human neuroblastoma cells exposed to KCl 120 mM, in which survival is tested 24 hours later.

### SUMMARY OF THE INVENTION

In one aspect the present invention provides a family of compounds of formula I, some of them present in the marine sponge *Aplysina cavernicola,* which block VDCC and inhibit AChE and BuChE and which are, consequently, useful for the treatment of brain ischemia, stroke, cognitive disorders as senile dementia, cerebrovascular dementia, mild recognition impairment, attention deficit disorder, and/or neurodegenerative dementing disease with aberrant protein aggregations as specially Alzheimers's disease or condition, or prion disease as Creutzfeld-Jacob disease or Gerstmann-Straussler-Scheinher disease.

In another aspect the present invention relates to the compounds of formula I for use as a medicament, in particular for the treatment of brain ischemia, stroke, cognitive disorders as senile dementia, cerebrovascular dementia, mild recognition impairment, attention deficit disorder, and/or neurodegenerative dementing disease with aberrant protein aggregations as specially Alzheimers's disease or condition, or prion disease as Creutzfeld-Jacob disease or Gerstmann-Straussler-Scheinher disease, and to the use of the compounds of formula I in the manufacture of a medicament, in particular for the treatment of the cognitive disorders mentioned above. A very related aspect of the invention is constituted by the pharmaceutical compositions comprising a compound of formula I.

A further aspect of the invention relates to processes either for preparing or for isolating from the marine sponge *Aplysina cavernicola* the compounds of formula I.

Finally, the present invention is also directed to organic solvent extracts of *Aplysina cavernicola*, to said extracts for use as medicaments, in particular for the treatment of cognitive disorders, to the use of said extracts in the manufacture of a medicament, in particular for the treatment of cognitive disorders, and to a process for obtaining said extracts.

### DETAILED DESCRIPTION OF THE INVENTION

Organic solvent extracts, in particular the isopropanolic extract, of the *Aplysina cavernicola* collected at Mediterranean sea, showed VDCC blocker activity (94% of inhibition at 50 µg/ml) and inhibit AChE and BuChE enzymes (41% and 27% of inhibition respectively at 25 µg/ml). The isopropanolic extract was partitioned between water and diethyl ether, and was the organic layer in which the inventors found the VDCC blocker activity and the inhibitory activity of AChE and BuChE enzymes. Those compounds that exhibited a neuroprotective effect were selected and further assayed in SH-SY5Y cells loaded with Fluo-4 to measure their effects on changes in intracellular Ca²⁺ in response to depolarization with KCl. The AChE and BuChE inhibitory activities of the compounds have also been evaluated following the Ellman's method (Ellman's et al., 1961, Biochem Pharmacol, 7, 88-95). All of them showed inhibition of these enzymatic activities in the 10⁻⁵ M range.

In conclusion, after careful investigations, the authors of the invention have found a family of compounds, some of them present in the marine sponge *Aplysina cavernicola,* that block VDCC and inhibit of AChE and BuChE. The combination of these properties could be a good approach in the treatment of AD.

The present invention provides, as stated above, a structurally distinct family of compounds of formula I, some of them present in the marine sponge *Aplysina cavernicola*, which block VDCC and inhibit of AChE and BuChE. Characteristic of the compounds is the presence of two heterocyclic units comprising a spiro ring connected through a linker of a length between a certain range. The compunds are represented by formula I: 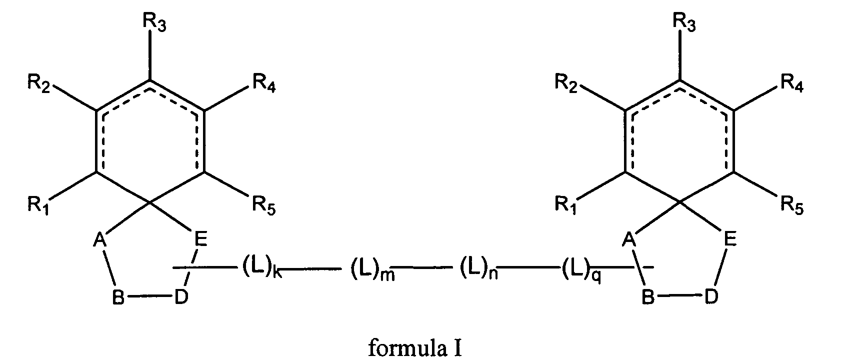 wherein
A, B, D and E are independently selected from =CR₆-, -CR₆R₇-, -CO-, -O-, -S-, -S(O)ₜ-, =N-, -N= or -NR₈-, provided that at least one of A, B, C or D is an heteroatom;
R₆, R₇ and R₈ are selected independently from hydrogen, substituted or unsubstituted alkyl, substituted or unsubstituted cycloalkyl, substituted or unsubstituted alkenyl, substituted or unsubstituted aryl, substituted or unsubstituted aralkyl, substituted or unsubstituted heterocyclyl, -CORₐ, -C(O)ORₐ, -OC(O)Rₐ,
-C(O)NRₐR_{b}, -C=NRₐ, -CN, -ORₐ, -S(O)ₜ-Rₐ, -NRₐR_{b}, -NRₐC(O)R_{b}, -NO₂, -N=CRₐR_{b} or halogen;
each L is independently selected from -CR₆R₇-,-CR₆= , -CO-, -O-, -S-, arylene, cycloalkylene, heterocyclylene, or -NR₈-;
k, m, n and q are each an integer independently selected from 0, 1, 2, 3, 4, 5, 6, 7, 9 or 10, provided the linker contains between 3 and 20 L units;
R₁ to R₅ are independently selected from hydrogen, substituted or unsubstituted alkyl, substituted or unsubstituted cycloalkyl, substituted or unsubstituted alkenyl, substituted or unsubstituted aryl, substituted or unsubstituted heterocyclyl, -CORₐ, -C(O)ORₐ, -OC(O)Rₐ, -C(O)NRₐR_{b}, -C=NRₐ, -CN, -ORₐ, -S(O)ₜ-Rₐ, -NRₐR_{b}, -NRₐC(O)R_{b}, -NO₂, -N=CRₐR_{b} or halogen and they can be the same or different in the two units connected by the linker;
Rₐ and R_{b} are each independently selected from hydrogen, substituted or unsubstituted alkyl, substituted or unsubstituted cycloalkyl, substituted or unsubstituted alkenyl, substituted or unsubstituted aryl, substituted or unsubstituted aralkyl, substituted or unsubstituted heterocyclyl, substituted or unsubstituted alkoxy, substituted or unsubstituted aryloxy or halogen;
t is 0,1 or 2;
the dotted line in the ring means that two double bonds can be present in the underlined part of said ring;
or a tautomer, a pharmaceutically acceptable salt, a prodrug or a solvate thereof; with the proviso that this compound is neither Aerothionin, nor Homoaerothionin nor 11,19-dideoxyfistularin, nor oxohomoaerothionin, nor oxoaerothionin nor 19-hydroxyfistularin-3; nor 19S-hydroxyfistularin-3-(R)-MTPA triester nor 19S-hydroxyfistularin-3-(S)-MTPA triester, nor 11,19-dihydroxyfistularin-3.

Preferably the linker contains one or more amide units of the type -CONRₐ- or -NRₐCO-, more preferably there are two units.

In a preferred embodiment A is -O- and B is -N=. In this case it is preferred that C is and D are -CR₆R₇-, most preferably -CH₂-.

In the above definition of compounds of formula (I) the following terms have the meaning indicated:
"Alkyl" refers to a straight or branched hydrocarbon chain radical consisting of carbon and hydrogen atoms, containing no saturation, having one to eight carbon atoms, and which is attached to the rest of the molecule by a single bond, e. g., methyl, ethyl, n-propyl, i-propyl, n-butyl, t-butyl, n-pentyl, etc. Alkyl radicals may be optionally substituted by one or more substituents such as a halo, hydroxy, alkoxy, carboxy, cyano, carbonyl, acyl, alkoxycarbonyl, amino, nitro, mercapto and alkylthio.
"Aryl" refers to a phenyl, naphthyl, indenyl, fenanthryl or anthracyl radical, preferably phenyl or naphthyl radical. The aryl radical may be optionally substituted by one or more substituents such as hydroxy, mercapto, halo, alkyl, phenyl, alkoxy, haloalkyl, nitro, cyano, dialkylamino, aminoalkyl, acyl and alkoxycarbonyl, as defined herein.
"Aralkyl" refers to an aryl group linked to an alkyl group. Preferred examples include benzyl and phenethyl.
"Cycloalkyl" refers to a stable 3-to 10-membered monocyclic or bicyclic radical which is saturated or partially saturated, and which consist solely of carbon and hydrogen atoms. Unless otherwise stated specifically in the specification, the term "cycloalkyl" is meant to include cycloalkyl radicals which are optionally substituted by one or more substituents such as alkyl, halo, hydroxy, amino, cyano, nitro, alkoxy, carboxy and alkoxycarbonyl.
"Halogen" refers to bromo, chloro, iodo or fluoro.
"Heterocyclyl" refers to a stable 3-to 15 membered ring radical which consists of carbon atoms and from one to five heteroatoms selected from the group consisting of nitrogen, oxygen, and sulfur, preferably a 4-to 8-membered ring with one or more heteroatoms, more preferably a 5-or 6-membered ring with one or more heteroatoms. For the purposes of this invention, the heterocycle may be a monocyclic, bicyclic or tricyclic ring system, which may include fused ring systems; and the nitrogen, carbon or sulfur atoms in the heterocyclyl radical may be optionally oxidised; the nitrogen atom may be optionally quaternized ; and the heterocyclyl radical may be partially or fully saturated or aromatic. Examples of such heterocycles include, but are not limited to, azepines, benzimidazole, benzothiazole, furan, isothiazole, imidazole, indole, piperidine, piperazine, purine, quinoline, thiadiazole, tetrahydrofuran.

The terms "arylene", "cycloalkylene" and "heterocyclylene" refer to aryl, cycloalkyl and cycloalkyl groups having two free radicals.

The dotted line in the ring means that two double bonds can be present in the underlined part of said ring.

References herein to substituted groups in the compounds of the present invention refer to the specified moiety that may be substituted at one or more available positions by one or more suitable groups, e. g., halogen such as fluoro, chloro, bromo and iodo ; cyano ; hydroxyl ; nitro ; azido ; alkanoyl such as aC1-6 alkanoyl group such as acyl and the like ; carboxamido ; alkyl groups including those groups having 1 to about 12 carbon atoms or from 1 to about 6 carbon atoms and more preferably 1-3 carbon atoms ; alkenyl and alkynyl groups including groups having one or more unsaturated linkages and from 2 to about 12 carbon or from 2 to about 6 carbon atoms ; alkoxy groups having one or more oxygen linkages and from 1 to about 12 carbon atoms or 1 to about 6 carbon atoms ; aryloxy such as phenoxy ; alkylthio groups including those moieties having one or more thioether linkages and from 1 to about 12 carbon atoms or from 1 to about 6 carbon atoms ; alkylsulfinyl groups including those moieties having one or more sulfinyl linkages and from 1 to about 12 carbon atoms or from 1 to about 6 carbon atoms ; alkylsulfonyl groups including those moieties having one or more sulfonyl linkages and from 1 to about 12 carbon atoms or from 1 to about 6 carbon atoms ; aminoalkyl groups such as groups having one or more N atoms and from 1 to about 12 carbon atoms or from 1 to about 6 carbon atoms ; carbocylic aryl having 6 or more carbons, particularly phenyl or naphthyl and aralkyl such as benzyl. Unless otherwise indicated, an optionally substituted group may have a substituent at each substitutable position of the group, and each substitution is independent of the other.

The term "pharmaceutically acceptable salts, derivatives, solvates , prodrugs" refers to any pharmaceutically acceptable salt, ester, solvate, or any other compound which, upon administration to the recipient is capable of providing (directly or indirectly) a compound as described herein. However, it will be appreciated that non-pharmaceutically acceptable salts also fall within the scope of the invention since those may be useful in the preparation of pharmaceutically acceptable salts. The preparation of salts, prodrugs and derivatives can be carried out by methods known in the art.

For instance, pharmaceutically acceptable salts of compounds provided herein are synthesized from the parent compound which contains a basic or acidic moiety by conventional chemical methods. Generally, such salts are, for example, prepared by reacting the free acid or base forms of these compounds with a stoichiometric amount of the appropriate base or acid in water or in an organic solvent or in a mixture of the two. Generally, nonaqueous media like ether, ethyl acetate, ethanol, isopropanol or acetonitrile are preferred. Examples of the acid addition salts include mineral acid addition salts such as, for example, hydrochloride, hydrobromide, hydroiodide, sulphate, nitrate, phosphate, and organic acid addition salts such as, for example, acetate, maleate, fumarate, citrate, oxalate, succinate, tartrate, malate, mandelate, methanesulphonate and p-toluenesulphonate. Examples of the alkali addition salts include inorganic salts such as, for example, sodium, potassium, calcium, ammonium, magnesium, aluminium and lithium salts, and organic alkali salts such as, for example, ethylenediamine, ethanolamine, N,N-dialkylenethanolamine, triethanolamine, glucamine and basic aminoacids salts.

Particularly favored derivatives or prodrugs are those that increase the bioavailability of the compounds of this invention when such compounds are administered to a patient (e.g., by allowing an orally administered compound to be more readily absorbed into the blood) or which enhance delivery of the parent compound to a biological compartment (e.g., the brain or lymphatic system) relative to the parent species.

Any compound that is a prodrug of a compound of formula (I) is within the scope of the invention. The term "prodrug" is used in its broadest sense and encompasses those derivatives that are converted in vivo to the compounds of the invention. Such derivatives would readily occur to those skilled in the art, and include, depending on the functional groups present in the molecule and without limitation, the following derivatives of the present compounds: esters, amino acid esters, phosphate esters, metal salts sulfonate esters, carbamates, and amides.

The compounds of the invention may be in crystalline form either as free compounds or as solvates and it is intended that both forms are within the scope of the present invention. Methods of solvation are generally known within the art. Suitable solvates are pharmaceutically acceptable solvates. In a particular embodiment the solvate is a hydrate.

The compounds of formula (I) or their salts or solvates are preferably in pharmaceutically acceptable or substantially pure form. By pharmaceutically acceptable form is meant, inter alia, having a pharmaceutically acceptable level of purity excluding normal pharmaceutical additives such as diluents and carriers, and including no material considered toxic at normal dosage levels. Purity levels for the drug substance are preferably above 50%, more preferably above 70%, most preferably above 90%. In a preferred embodiment it is above 95% of the compound of formula (I), or of its salts, solvates or prodrugs.
The compounds of the present invention represented by the above described formula (I) may include enantiomers depending on the presence of chiral centres or isomers depending on the presence of multiple bonds (e.g. Z, E). The single isomers, enantiomers or diastereoisomers and mixtures thereof fall within the scope of the present invention.

The compounds Aerothionin, Homoaerothionin and 11,19-dideoxyfistularin are represented by formulae Ia, Ib and Ic:

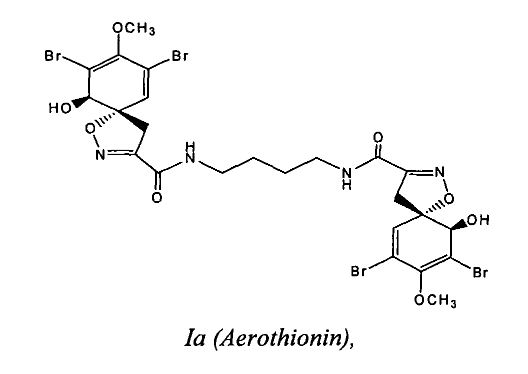

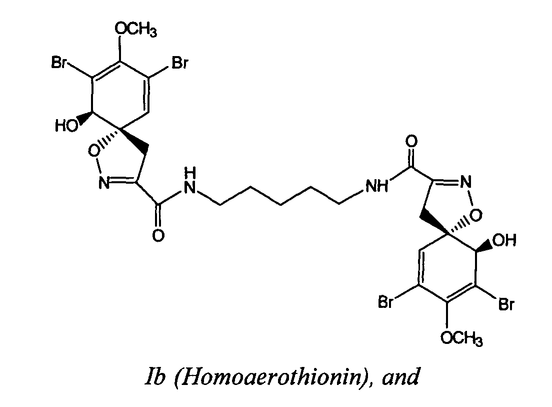

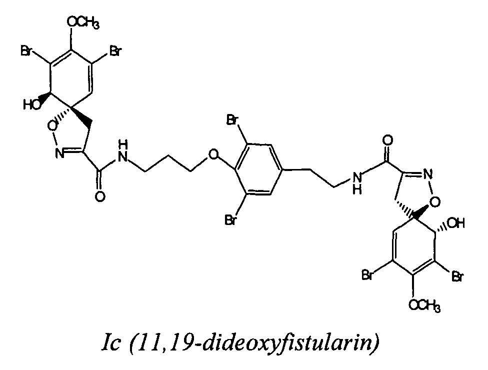

The compound of formula Ia was described by L. Minale and G. Sodano. J. in *J*. *Chem.Soc.Chem. Commun,* 0, 1970, 751-753; whereas compound of formula Ib was described in *J.Chem.Soc.Perkin Trans,* 0 ,18-24, 1972 and the compound of formula Ic was described by M.R. Kernan, R.C. Camhie in *J. Nat. Prod.* 1990,53,615-622. None of these documents discloses or suggests that these compounds presented VDCC blocker activity with additional AChE and butyrylcholinesterase (BuChE) inhibitory activities.

An aspect of the present invention relates to the compounds of formula I for use as a medicament, in particular for their use in the treatment of cognitive disorders as senile dementia, cerebrovascular dementia, mild recognition impairment, attention deficit disorder, and/or neurodegenerative dementing disease with aberrant protein aggregations as specially Alzheimers's disease or condition, stroke or prion disease as Creutzfeld-Jacob disease or Gerstmann-Straussler-Scheinher disease.

A further aspect of the invention relates to the use of the compounds of formula I in the manufacture of a medicament. In particular for the manufacture of a medicament for the treatment of the cognitive disorders mentioned above.

The present invention further provides pharmaceutical compositions comprising a compound of this invention, or a pharmaceutically acceptable salt, derivative, prodrug or stereoisomers thereof together with a pharmaceutically acceptable carrier, adjuvant, or vehicle, for administration to a patient.
Examples of pharmaceutical compositions include any solid (tablets, pills, capsules, granules etc.) or liquid (solutions, suspensions or emulsions) composition for oral, topical or parenteral administration.

In a preferred embodiment the pharmaceutical compositions are in oral form, either solid or liquid. Suitable dose forms for oral administration may be tablets, capsules, syrops or solutions and may contain conventional excipients known in the art such as binding agents, for example syrup, acacia, gelatin, sorbitol, tragacanth, or polyvinylpyrrolidone; fillers, for example lactose, sugar, maize starch, calcium phosphate, sorbitol or glycine; tabletting lubricants, for example magnesium stearate; disintegrants, for example starch, polyvinylpyrrolidone, sodium starch glycollate or microcrystalline cellulose; or pharmaceutically acceptable wetting agents such as sodium lauryl sulfate.

The solid oral compositions may be prepared by conventional methods of blending, filling or tabletting. Repeated blending operations may be used to distribute the active agent throughout those compositions employing large quantities of fillers. Such operations are conventional in the art. The tablets may for example be prepared by wet or dry granulation and optionally coated according to methods well known in normal pharmaceutical practice, in particular with an enteric coating.

The pharmaceutical compositons may also be adapted for parenteral administration, such as sterile solutions, suspensions or lyophilized products in the apropriate unit dosage form. Adequate excipients can be used, such as bulking agents, buffering agents or surfactants.

The mentioned formulations will be prepared using standard methods such as those described or referred to in the Spanish and US Pharmacopoeias and similar reference texts.

Administration of the compounds or compositions of the present invention may be by any suitable method, such as intravenous infusion, oral preparations, and intraperitoneal and intravenous administration. Oral administration is preferred because of the convenience for the patient and the chronic character of the diseases to be treated.

Generally an effective administered amount of a compound of the invention will depend on the relative efficacy of the compound chosen, the severity of the disorder being treated and the weight of the sufferer. However, active compounds will typically be administered once or more times a day for example 1, 2, 3 or 4 times daily, with typical total daily doses in the range of from 0.1 to 1000 mg/kg/day. It will be appreciated that it may be necessary to make routine variations to the dosage, depending on the age and condition of the patient, and the route of administration.

The compounds and compositions of this invention may be used with other drugs to provide a combination therapy. The other drugs may form part of the same composition, or be provided as a separate composition for administration at the same time or at different time.

The compounds of formula (I) defined above can be obtained by a convergent pathway strategy by coupling the two heterocyclic moieties which contain part of the linker. Synthetic procedures for preparing the compounds of formula (I) are described by Wasserman, H. et al. in *J.Org. Chem*. 1998, 63,5581-5586, and by Nishiyama S. et al. in *Tetrahedron Letters,* Vol.24, No. 32, pp. 3351-3352, 1983. The person skilled in the art of organic synthesis will readily design the process for each compound depending on the desired functionality of the heterocycles, the substituents and the nature of the linker to be obtained.

The reaction products may, if desired, be purified by conventional methods, such as crystallisation or chromatography. Where the above described processes for the preparation of compounds of the invention give rise to mixtures of stereoisomers, these isomers may be separated by conventional techniques such as preparative chromatography. If there are chiral centers the compounds may be prepared in racemic form, or individual enantiomers may be prepared either by enantiospecific synthesis or by resolution.

One preferred pharmaceutically acceptable form is the crystalline form, including such form in a pharmaceutical composition. In the case of salts and solvates the additional ionic and solvent moieties must also be non-toxic. The compounds of the invention may present different polymorphic forms, it is intended that the invention encompasses all such forms.

The compounds of formula (I) defined above can also be obtained by isolation of the compound from the marine sponge Aplysina cavernicola, according to the following process which comprises the following steps:
a) effecting an extraction of a previously triturated *Aplysina cavernicola* with an organic solvent, in particular isopropanol
b) optionally concentrating the organic solvent extract and effecting a water/ether extraction of the product obtained in step a), and
c) effecting a column chromatography of the product obtained in the previous step in order to isolate the individual compounds.

The invention is also directed to organic solvent extracts of *Aplysina cavernicola;* to said extracts for use as medicaments, in particular for the treatment of cognitive disorders as senile dementia, cerebrovascular dementia, mild recognition impairment, attention deficit disorder, and/or neurodegenerative dementing disease with aberrant protein aggregations as specially Alzheimers's disease or condition, stroke or prion disease as Creutzfeld-Jacob disease or Gerstmann-Straussler-Scheinher disease; to the use of said extracts in the manufacture of a medicament, in particular for the manufacture of a medicament for the treatment of brain ischemia, stroke, cognitive disorders as senile dementia, cerebrovascular dementia, mild recognition impairment, attention deficit disorder, and/or neurodegenerative dementing disease with aberrant protein aggregations as specially Alzheimers's disease or condition, or prion disease as Creutzfeld-Jacob disease or Gerstmann-Straussler-Scheinher disease; and, finally, to a process for obtaining said extracts which comprises effecting an extraction of a previously triturated *Aplysina* *cavernicola* with an organic solvent, in particular isopropanol. The process preferably further comprises the consisting of concentrating the organic solvent extract and effecting a water/ether extraction of the product obtained in the previous step.

The following examples are given as further illustration of the invention, they should not be taken as a definition of the limits of the invention.

### EXAMPLES

The general procedures for the preparation of compounds of the invention have been described above.

### Example 1: Isolation

### 1. Animal material:

The sponge was collected in 2001 at Ibiza sea (Spain). The sponge forms irregular straplike branches, the surface is conculose with fine protruding fibers, and it is flexible and very difficult to tear. The sponge is deep brownish-yellow in life and cream in preservative.

### 2. Extraction and Isolation :

A sample of frozen sponge (600 gr wet weight) was cut into small pieces (2 cm) and triturated, the marine material was extracted with isopropanol (3L x 2). After decantation, the combined extracts were concentrated to give a brown solid (12 gr) and partitioned between diethyleter and water. The organic layer was concentrated to give a brown oil (1.67 gr). The oil was chromatographed on silica gel with a stepwise gradient solvent system: DCM/ MeOH (100:1), DCM/ MeOH (75:1), DCM/ MeOH (50:1), DCM/ MeOH (25:1). After this chromatography purification we isolated five compounds and all of them were described as Aeroplysinin-2 (compound 1), Aeroplysinin-1 (compound 2), Aerothionin (compound 3), Homoaerothionin (compound 4), and 11,19-dideoxyfistularin (compound 5).

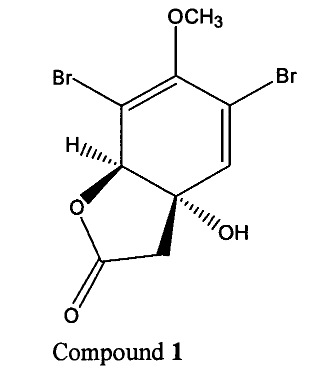

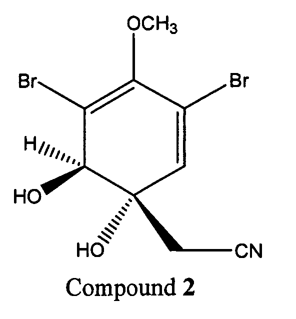

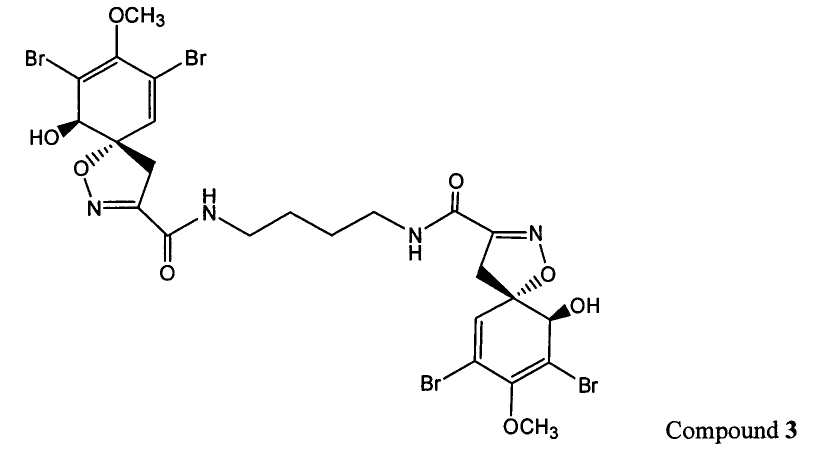

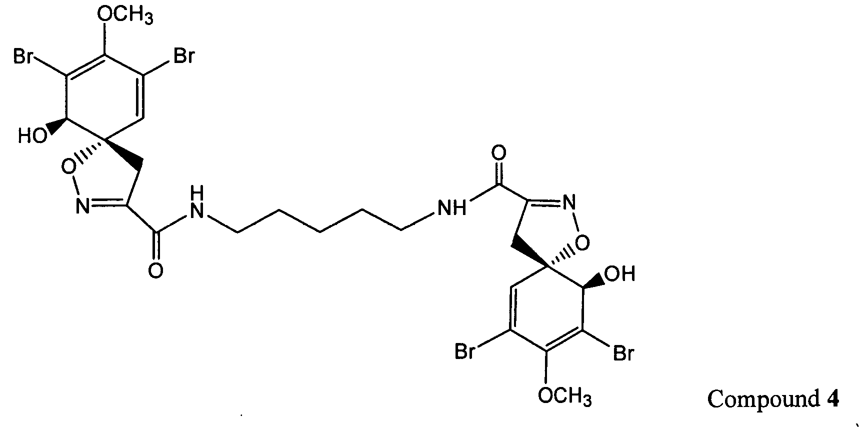

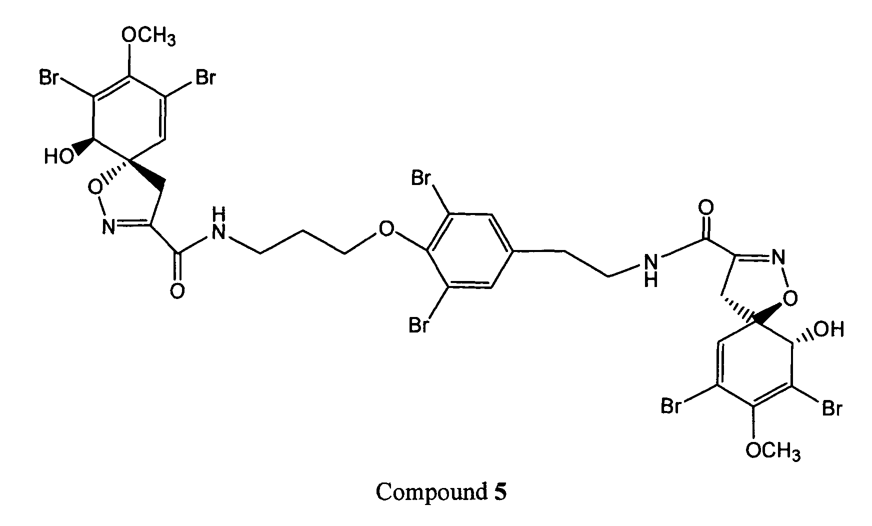

The compound **1** was described by L. Minale and G. Sodano. *J. Chem.Soc.Chem. Commun,* 0, 1972, 674-675, the Compound **2** and **3** were described by the same authors in *J Chem.Soc.Chem. Commun,* 0, 1970, 751-753. The Compounds **4** was described in *J.Chem.Soc.Perkin Trans,* 0 ,18-24, 1972 and the compound **5** was described in M.R. Kernan, R.C. Camhie . *J. Nat. Prod*. 1990,53,615-622.

### 3. Materials and methods:

HPLC was perform with a symmetry C18 (4.6x150mm, 3.5µm) column using a Waters Alliance 2695 with a 2996 photodiode array detector and Waters ZQ2000 mass spectrometer used for the analytical separation and for UV and mass determination. The gradient used for the elution was 0-5 min 80 % A: 20%B; 5-40 min 100%B; 40-45 min 100%B; 45-46 min 80%A: 20%B (A= H2O 0.1% HF; B=AcN 0.1%HF).
IR Spectra were recorded on a Bruker Tensor 27 FT-IR spectrometer.
¹H, ¹³C, NMR spectra were recorded on a Varian Mercury 400 MHz spectrometer in CD₃OD using the solvent as a reference standard (¹H, 4.87, 3.31, and ¹³C, 49.1). ¹H, ¹³C, COSY, HSQC, HMBC, NOE and DEPT spectra were obtained using standard Varian pulse sequences.

### Example 2: Biological Activity

### 1. Effects on VDCC

In order to found new marine compounds with VDCC blocker activity, we have performed a screening on SH-SY5Y neuroblastoma cells exposed to KCl 120 mM, The ability to block calcium channels is evaluated measuring cellular viability, using the LDH assay. Previously to the depolarization these cells were pretreated for 1 hour with the marine extracts at different concentrations (50, 15 and 5 µg/ml), 24 hours later survival is tested. Those compounds that exhibited a neuroprotective effect were selected and further assayed in SH-SY5Y cells loaded with Fluo-4 (Molecular probes) to measure their effects on changes in intracellular Ca²⁺ in response to depolarization with 60 mM KCl.
SH-SY5Y cells were plated at 5x10⁵ cells per well into Black / Clear Bottom 96-well culture plate, 48 hours before treatment. Cells were loaded with Fluo-4, 5 µM and pluronic acid, 0.1%, for 30 min at 37°C, 5%CO₂, following a incubation of 15 min at RT in Krebs-HEPES solution. Immediately cells are exposed to the samples at different concentrations for10 min. The marine extracts were tested, in Krebs-HEPES, at final concentrations of 50 µg/ml, 15 µg/ml and 5 µg/ml, and the isolated compounds at 10⁻⁵, 10⁻⁶ and 5x10⁻⁸ M. After the treatment, the fluorescence is measured in a Fluostar Optima plate reader (BMG) in response to depolarization with 60 mM KCl. The excitation wavelength was 485 nm, and that of emission 520 nm
The marine extract from the sponge *Aplysina cavernicola* showed neuroprotective activity and VDCC blocker activity. Three compounds isolated from this extract have shown calcium channel blocking properties in SH-SY5Y neuroblastoma cells. Results are showed in table 1.

### 2. AChE Inhibition

AChE inhibitory activity was evaluated at 30°C by the colorimetric method reported by Ellman (Ellman, G.L. et al., 1961, *Biochem Pharmacol, 7*, 88-95). The assay solution consisted of 0.1 M phosphate buffer pH 8, 0.3 mM 5,5'-dithiobis (2-nitrobenzoic acid) (DTNB, Ellman's reagent), 0.02 units AChE (Sigma Chemical Co. from bovine erythrocytes), and 0.5 mM acetylthiocholine iodide as the substrate of the enzymatic reaction. The compounds tested were added to the assay solution and pre incubated with the enzyme for 5 min at 30°C. After that period, the substrate was added. The absorbance changes at 405 nm were recorded for 5 min with a microplate reader Digiscan 340T, the reaction rates were compared, and the percent inhibition due to the presence of test compounds was calculated. The reaction rate was calculated with, at least, triplicate measurements, and the percent inhibition due to the presence of test compound was calculated relative to the compound-free control. The results are shown in table 1.

### 3. BuChE inhibition

BuChE inhibitory activity was evaluated at 30°C by the colorimetric method reported by Ellman (Ellman, G.L. et al., 1961, *Biochem Pharmacol,* 7, 88-95). The assay solution consisted of 0.01 units BuChE from human serum, 0.1 M sodium phosphate buffer pH 8, 0.3 mM 5,5'-dithiobis (2-nitrobenzoic acid) (DTNB, Ellman's reagent), and 0.5 mM butyrylthiocholine iodide as the substrate of the enzymatic reaction. Enzyme activity was determined by measuring the absorbance at 405 nm during 5 minutes with a microplate reader Digiscan 340T. The tested compounds were preincubated with the enzyme for 10 minutes at 30°C. The reaction rate was calculated with, at least, triplicate measurements. The results are shown in table 1.

### 4. Toxicity measurement

The cytotoxicity effect of the molecules was tested in the human neuroblastoma cell line SH-SY5Y. These cells were cultured in 96-well plates in minimum essential medium, Ham's F12 medium, supplemented with 10% fetal bovine serum, 1% glutamine and 1% penicillin/streptomycin, and grown in a 5% CO₂ humidified incubator at 37°C. Cells were plated at 10⁴ cells for each well, at least, 48 hours before treatment. Cells were exposed for 24 hours to the compounds at different concentrations (from 10⁻⁵ to 10⁻⁹), quantitative assessment of cell death was made by measurement of the intracellular enzyme lactate dehydrogenase (LDH) (citotoxicity detection kit, Roche). The cuantity of LDH was measured was evaluated in a microplate reader Anthos 2010, at 492 and 620 nm. Controls were taken as 100% viability. The results are shown in table 1.

### 5. Propidium competition

Propidium exhibits an increase in fluorescence on binding to AChE peripheral site, making it a useful probe for competitive ligand binding to the enzyme. Fluorescence was measured in a Fluostar Optima plate reader (BMG). Measurements were carried out in 100 µl solution volume, in 96-well plates. The buffer used was 1 mM Tris/HCl, pH 8.0. 5 µM AChE was incubated, at least 6 hours, with the molecule at different concentrations. 20 µM propidium iodide was added 10 min before fluorescence measurement. The excitation wavelength was 485 nm and that of emission 620 nm. *In vitro* results are showed in table 1.

### 6. Antioxidant activity

In order to evaluate if these compounds have antioxidant properties, cells SH-SY5Y were exposed 24 hours to 100 µM H₂O₂, previously these cells were pretreated for 1 hour with the molecules at different concentrations (from 10⁻⁵ to 10⁻⁹). The antioxidant activity is evaluated measuring cellular viability, using the LDH assay. Cells SH-SY5Y were cultured in 96-well plates as toxicity experiments. The results are showed in table 1.

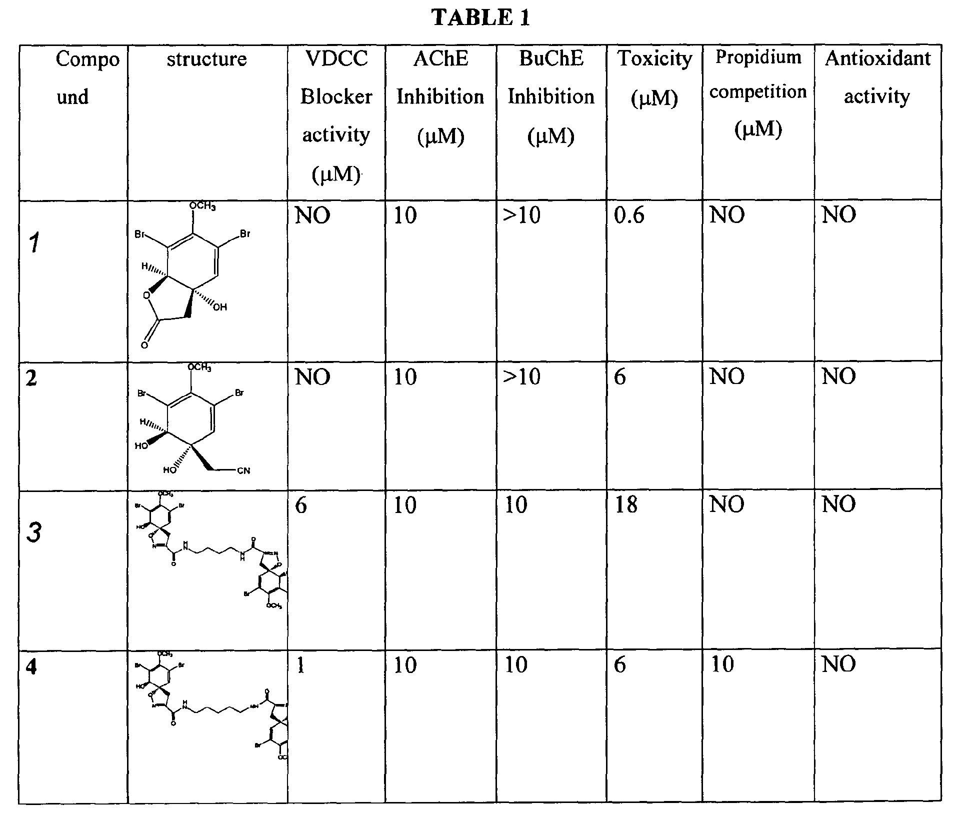

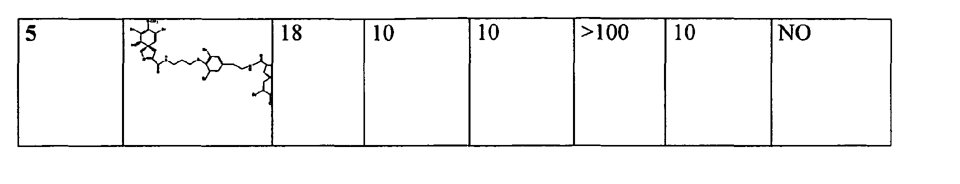

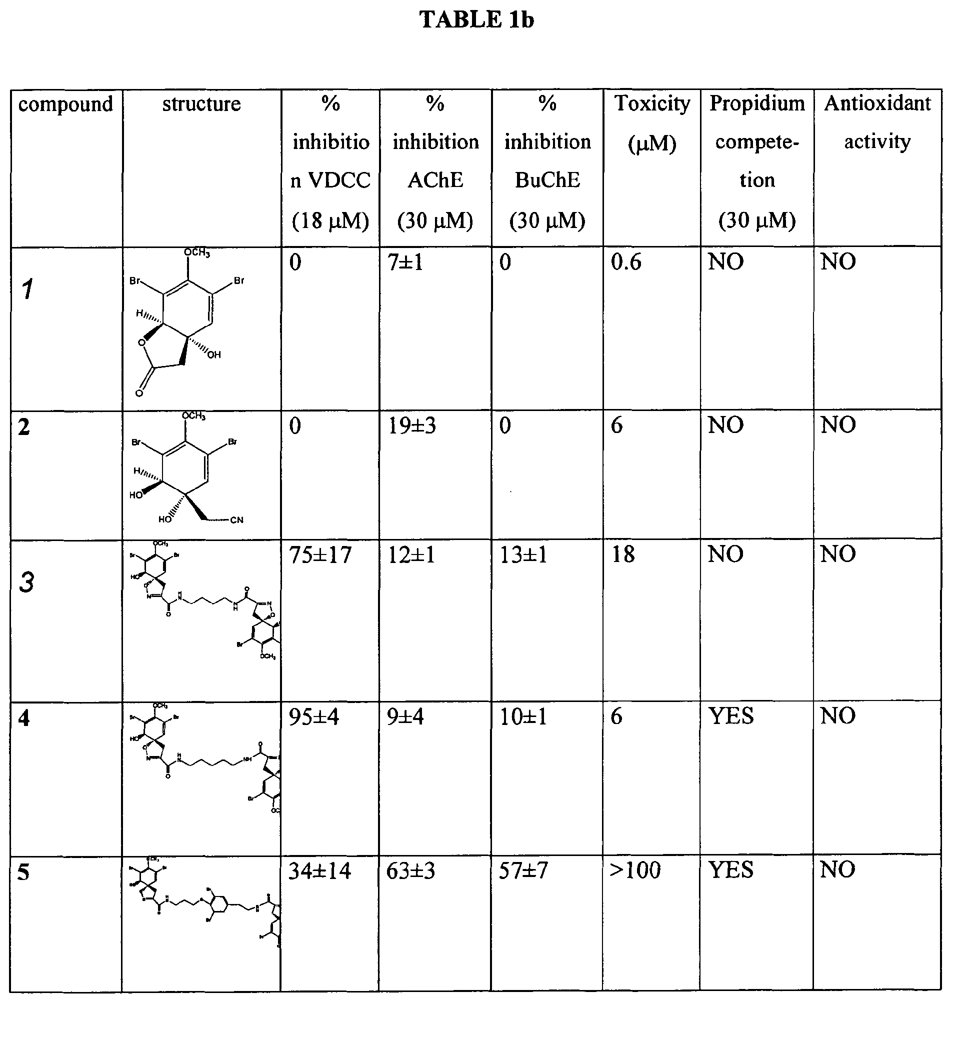

## Claims

1. A compound of formula I: 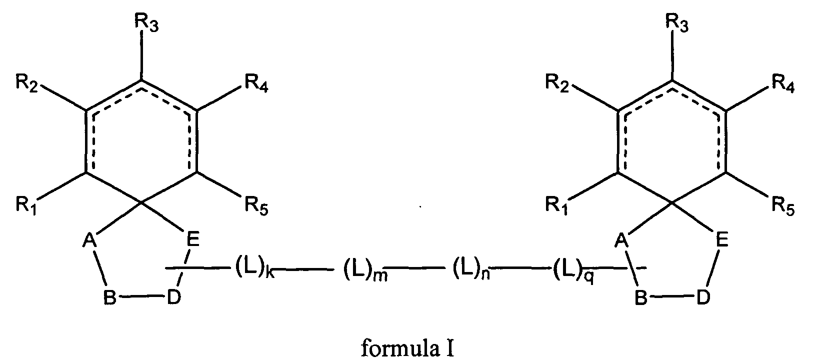 wherein
A, B, D and E are independently selected from =CR₆-, -CR₆R₇-, -CO-, -O-, -S-, -S(O)ₜ-, =N-, -N= or -NR₈-, provided that at least one of A, B, C or D is an heteroatom;
R₆, R₇ and R₈ are selected independently from hydrogen, substituted or unsubstituted alkyl, substituted or unsubstituted cycloalkyl, substituted or unsubstituted alkenyl, substituted or unsubstituted aryl, substituted or unsubstituted aralkyl, substituted or unsubstituted heterocyclyl, -CORₐ, -C(O)ORₐ, -OC(O)Rₐ,
-C(O)NRₐR_{b}, -C=NRₐ, -CN, -ORₐ, -S(O)ₜ-Rₐ, -NRₐR_{b}, -NRₐC(O)R_{b}, -NO₂, -N=CRₐR_{b} or halogen;
each L is independently selected from -CR₆R₇-,-CR₆= , -CO-, -O-, -S-, arylene, cycloalkylene, heterocyclylene, or -NR₈-;
k, m, n and q are each an integer independently selected from 0, 1, 2, 3, 4, 5, 6, 7, 9 or 10, provided the linker contains between 3 and 20 L units;
R₁ to R₅ are independently selected from hydrogen, substituted or unsubstituted alkyl, substituted or unsubstituted cycloalkyl, substituted or unsubstituted alkenyl, substituted or unsubstituted aryl, substituted or unsubstituted heterocyclyl, -CORₐ,
-C(O)ORₐ, -OC(O)Rₐ, -C(O)NRₐR_{b}, -C=NRₐ, -CN, -ORₐ, -S(O)ₜ-Rₐ, -NRₐR_{b}, -NRₐC(O)R_{b}, -NO₂, -N=CRₐR_{b} or halogen and they can be the same or different in the two units connected by the linker;
Rₐ and R_{b} are each independently selected from hydrogen, substituted or unsubstituted alkyl, substituted or unsubstituted cycloalkyl, substituted or unsubstituted alkenyl, substituted or unsubstituted aryl, substituted or unsubstituted aralkyl, substituted or unsubstituted heterocyclyl, substituted or unsubstituted alkoxy, substituted or unsubstituted aryloxy or halogen;
t is 0,1 or 2;
the dotted line in the ring means that two double bonds can be present in the underlined part of said ring;
or a tautomer, a pharmaceutically acceptable salt, a prodrug or a solvate thereof; with the proviso that this compound is neither Aerothionin, nor Homoaerothionin nor 11,19-dideoxyfistularin nor oxohomoaerothionin, nor oxoaerothionin nor 19-hydroxyfistularin-3, nor 19S-hydroxyfistularin-3-(R)-MTPA triester, nor 19S-hydroxyfistularin-3-(S)-MTPA triester, nor 11,19-dihydroxyfistularin-3.

2. Use of a compound as defined by formula I, or a tautomer, a pharmaceutically acceptable salt, a prodrug or a solvate thereof, in the manufacture of a medicament; preferably for the treatment of brain ischemia, stroke, cognitive disorders as senile dementia, cerebrovascular dementia, mild recognition impairment, attention deficit disorder, and/or neurodegenerative dementing disease with aberrant protein aggregations as specially Alzheimers's disease or condition, or prion disease as Creutzfeld-Jacob disease or Gerstmann-Straussler-Scheinher disease.

3. Use according to claim 2 wherein in the compound of formula I: A means: -O- and B means: -N=.

4. Use according to claim 3 wherein both D and E mean -CH₂-.

5. Use according to any of claims 2-4 wherein the linker that connects the two spirocyclic units comprises one or more units selected from -CONRₐ- or -NRₐCO-.

6. Use according to any of claims 2-5 wherein the linker -(L)ₖ-(L)ₘ-(L)ₗ-(L)_{q}- means: -CO-NH-(L)_{y}-NH-CO-, wherein y is selected from 2, 3, 4, 5, 6, 7, 8, 9 or 10.

7. Use according to claims 2-6 wherein the compound of formula I is selected from the group formed by the compounds:

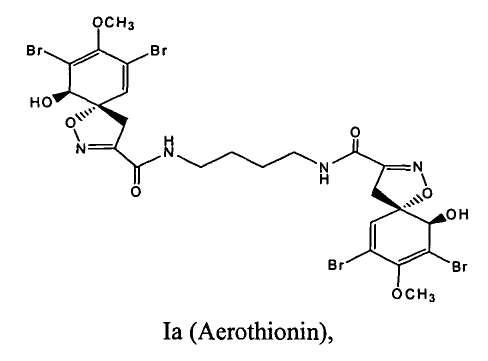

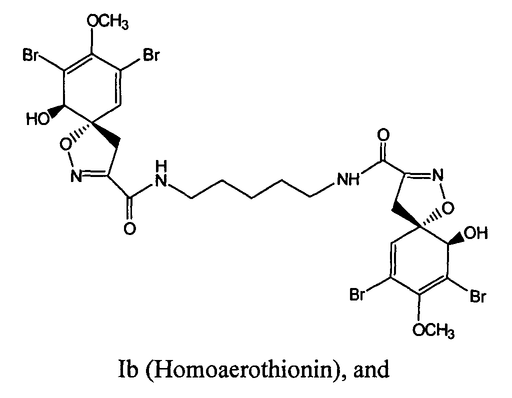

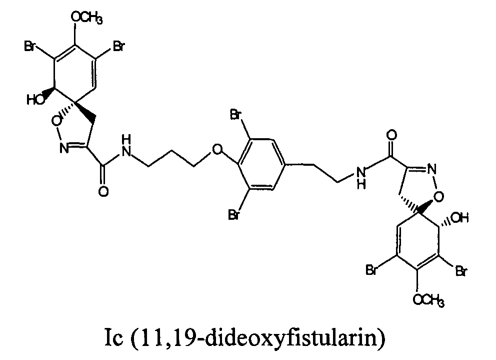

8. A pharmaceutical composition which comprises a compound as defined by formula I, or a tautomer, a pharmaceutically acceptable salt, a prodrug or a solvate thereof, together with a pharmaceutically acceptable carrier, adjuvant or vehicle.

9. A process for the isolation of a compound as defined in claim 1 which comprises the following steps:
d) effecting an extraction of a previously triturated *Aplysina cavernicola* with an organic solvent, in particular isopropanol
e) optionally concentrating the organic solvent extract and effecting a water/ether extraction of the product obtained in step a), and
f) effecting a column chromatography of the product obtained in the previous step in order to isolate the individual compounds.
